Europäisches Patentamt

European Patent Office

Office européen des brevets

(19)

(11) Publication number: **0 090 660**
A2

# (12) EUROPEAN PATENT APPLICATION

(21) Application number: **83301813.8**

(51) Int. Cl.³: **A 61 K 39/095**

(22) Date of filing: **30.03.83**

---

(30) Priority: **30.03.82 US 363480**

(43) Date of publication of application: **05.10.83**
**Bulletin 83/40**

(84) Designated Contracting States: **BE CH DE FR GB IT LI NL SE**

(71) Applicant: **THE GOVERNMENT OF THE UNITED STATES OF AMERICA as represented by the Secretary Department of Commerce, Washington, D.C. (US)**

(72) Inventor: **Buchanan, Thomas Mark, 702 32nd Ave South, Seattle, WA. 98144 (US)**
Inventor: **Olsen, Duane Arvid, 3335 Bridgeport Way West, Tacoma, WA. 98466 (US)**

(74) Representative: **Ruffles, Graham Keith et al, MARKS & CLERK 57-60 Lincoln's Inn Fields, London WC2A 3LS (GB)**

(54) **Neisseria gonorrhoeae vaccine.**

(57) A highly antigenic vaccine for protecting humans against infection by Neisseria gonorrhoeae microorganism containing one or more serotypes of protein I results from a method of preparing highly purified serotypes in high concentration and to increased solubility of the protein I serotypes.

# NEISSERIA GONORRHOEAE VACCINE

## BACKGROUND OF THE INVENTION

### Field Of The Invention

The present invention relates to an improved method of separating and purifying protein I from the principal outer membrane of Neisseria gonorrhoeae (N.g.) and a prophylactic vaccine produced therefrom. Specifically, the invention is directed to a method of preparing individual serotypes of N.g. protein I in higher yield and greater antigenicity and the production of a vaccine containing one or more serotypes capable of eliciting a bactericidal response in humans, which serotypes are present in significantly higher concentration than previously attainable.

### Description Of The Prior Art

Gonorrhea is an extremely common infection in humans and, in the United States alone, approximately four million persons annually appear in clinics and physicians' offices with this sexually-transmitted disease. Despite effective antibiotics, the annual number of cases of gonorrhea in the U.S. has remained essentially unchanged for the past five years. Some gonococci have become resistant to treatment with penicillin, which formerly was one of the most frequently employed medicaments, and there is a growing need to contain this contagion. Important complications of gonorrhea are gonococcal salpingitis or pelvic inflammatory disease (P.I.D.) which occurs in approximately 17% of women with the disease, and disseminated gonococcal infection (D.G.I.) which develops in 1 to 3% of all cases. Gonococcal salpingitis produces sterility in an estimated 50,000 women annually in the U.S. alone, and some people die each year from the heart valve infections that may accompany D.G.I. The Center for Disease Control, Atlanta, Georgia, has recently estimated that the health cost of these two (2) complications of gonorrhea exceeds 850 million dollars annually in the U.S. alone.

Heretofore, no successful vaccine has been available having efficacy against the numerous strains of N.g. There is evidence that P.I.D. infection with a strain of N.g. of a given protein I serotype protects against recurrent salpingitis with N.g. of the same protein I serotype (Buchanan, T.M., et al., American Journal Obstetrics & Gynecology, 138:978-980, December, 1980). In addition, D.G.I. is caused by a single protein I serotype of gonococci approximately 85-90% of the time (Buchanan, T.M. and Hildebrandt, J.F., Infection and Immunity, 32:985-994, June, 1981), and almost never recurs in the same patient. These data have indicated that a vaccine comprised of protein I of the correct single serotype should protect against D.G.I., and that a vaccine comprised of protein I of a sufficient number of serotypes (i.e., five) should protect against most P.I.D. due to N.g. A method of separating and purifying protein I of N.g. from toxic lipopolysaccharide (L.P.S.), which is found in the principal outer membrane (POMP) of N.g. along with other protein fragments, and a vaccine prepared therefrom is described by Buchanan in U.S. Patents 4,203,971 and 4,239,749, incorporated by reference herein. While this development was an important advancement in the field, the yields of protein I produced thereby are low, and, more importantly, the solubility of the protein I product is generally too low to produce a vaccine preparation that stimulates formation of protective antibodies in effective amounts, even though it is safe and non-toxic.

## SUMMARY OF THE INVENTION

The present invention relates to a method of producing N.g. protein I in higher yields than known heretofore, with no loss of antigenicity or increase in toxicity. The incorporation of homogenization and extraction steps with a chelating agent and suitable extracting agent results in an increase in yields on the order of 50 to 100 fold.

The instant invention also relates to a vaccine which is more immunogenic than any previous vaccine prepared from protein I of N.g. This results from another aspect of the invention, an improved technique of solubilizing purified protein I. The ability to prepare protein I in soluble form means that vaccines with significantly higher antigenicity and immunogenicity may be prepared since a given volume of vaccine contains greater amounts of protein. Thus, vaccines prepared according to the present invention may contain concentrations of protein on the order of 5 to 10 mg/ml, as compared to vaccines prepared in accordance with the disclosures in U.S. Patents 4,203,971 and 4,239,749, in which vaccines having concentrations on the order of 0.10 to 0.15 mg/ml are produced. Thus, the improved solubility of the protein I resulting from the method of the present invention results in approximately a 100 fold increase in antigenicity or potency of a sterile filtered protein I vaccine preparation. Specifically, the solubilization procedure involves the steps of (a) sonication of purified protein I in the presence of an appropriate solvent and detergent, (b) dialysis of the solution against the solvent at elevated pH to remove the detergent, and (c) filtration of the purified solution.

The present invention further relates to a vaccine which combines a plurality of serotypes of protein I. Human dosage required to produce an effective immune response is approximately 0.1 to 0.2 mg/serotype. Since protection against gonococcal P.I.D. and D.G.I. produced by protein I vaccines is serotype specific, it is necessary to combine many or most of the 10 different known serotypes of protein I, in order to produce a vaccine that would provide protection against most N.g. If the 10 serotypes of protein I each had a concentration of 5 mg/ml protein and were combined, the final vaccine would contain 0.5 mg/ml per serotype. At a human dosage of 0.2 mg per serotype, a volume of 0.4 ml would be required for the injection. This is an acceptable volume to inject into the subcutaneous tissue of the human arm. With the previous concentrations of soluble protein I being only about 0.1 mg/ml, a volume of 2 ml would be required for each serotype dosage of 0.2 mg, or a 20 ml volume for 10 serotypes. Since no more than 1 ml can be comfortably injected in the human arm, a protein I vaccine containing several serotypes was impractical prior to the instant invention.

Thus, the present invention affords broad protection against a number of different N.g. caused afflictions as a result of the improved method of purification, solubilization, and combination of from 3 to 9 serotypes of N.g. protein I.

## DESCRIPTION OF THE PREFERRED EMBODIMENTS

A. Preparation And Extraction Procedure

Neisseria gonorrhoeae are grown as disclosed in U.S. Patents 4,203,971 and 4,239,749. Basically, the procedure involves growth on standard solid or liquid media at $35.5°-37°C$ under increased $CO_2$ (2-15%, depending upon the strain of gonococci) for 16 to 24 hours, until the culture has completed log phase growth and has yielded near the maximum number of organisms for the culture conditions. The original gonococci used to seed the cultures are chosen to represent a single protein I serotype, and the colonial morphology of the organisms selected for each serotype is one that correlates with the best growth characteristics and outer membrane protein production to provide maximum yields of protein I (subunit m.w. 34,000-39,000) for the resulting vaccine. In general, this corresponds to colony types 3 or 4 which are fastest growing (Kellogg, D.S., Jr., et al., J. Bacteriology, 85:1274-1279, May, 1963), and transparent organisms (Swanson, J., "Studies on Gonococcus Infection. XII Colony Color and Opacity Variants of Gonococci." Infect. Immun., 19:320-331, January, 1978) which provide maximum yields of protein I and minimal amounts of other outer membrane proteins to contaminate the protein I during the purification procedure. Protein I of each serotype desired for the vaccine is prepared, purified, and characterized separately as described below.

Organisms are harvested at the end of their growth in culture medium by scraping the organisms from the culture plate and suspending them in ice cold saline (for solid medium only), followed by centrifugation to pellet the organisms away from the saline (solid medium) or liquid culture medium.

The increased yields of protein I, according to the present invention, are obtained by homogenizing the pellet of organisms, which results from centrifugation, that has been resuspended in liquid (0.1 g weight of organisms per ml of liquid) containing a compound which may be considered a solvent, solubilizing agent, or extracting agent; and a chelating agent.

The requirements of the extracting agent are an organic compound, preferably acyclic, containing at least one amino group and at least one hydroxyl group. Examples of compounds which are considered to be effective are ethanolamine, diethanolamine, triethanolamine, 2-amino-1-propanol, 2-amino-1,3-propanediol, and TRIS (tris (hydroxymethyl) aminomethane). Both 2-aminoethanol and TRIS are preferred with the former being most preferred.

The extracting agent is employed generally in the range of 0.05 to 0.6M. When TRIS is employed, a range of 0.15 to 0.25M is preferred and 0.2M is most preferred, corresponding to a pH of approximately 7.5. When ethanolamine is the solvent of choice, a concentration of 0.1 to 0.2M is quite suitable, 0.15 to 0.19M being preferred and 0.17M being most preferred.

The extracting agent employed in the present invention, although not confirmed, appears to function by deaggregating the protein I fragments formed in the homogenization process by complexing the protein. That is, each protein fragment appears to have one or more hydrophillic portions which are attracted to water and one or more hydrophobic portions which form non-covalent bonds or complexes. Thus, the extracting agent appears to separate and improve water solubility of the protein I fragments.

It is to be understood that the preceding explanation of the manner in which the extracting agent functions is theoretical and merely attempts to account, in part, for the unexpected results of the present invention. However, neither the applicants nor the present invention are to be limited or restricted by this theory.

The other reagent employed in the homogenization step is a chelating agent, which serves to bind divalent cations, thereby destabilizing the gonococcal outer membrane. The chelating agent also minimizes foaming of the organism suspension during homogenization, thus permitting higher homogenization speeds and more efficient homogenization of the organism outer membranes. Ethylenediaminetetraacetic acid (EDTA) and its salts are preferred as chelating agents. Specifically, the di- and tetra-sodium salts are preferred as chelating agents, in part, because of the suitable pH range which they provide. A suitable concentration range for the chelating agent is between 0.005M and 0.05M, preferably between 0.01M and 0.035M, with the most preferred concentration for disodium EDTA being 0.02M.

The organisms are suspended during homogenization at a rate of about 0.02 to 0.3 g wet weight of organisms/ml of solution, 0.075 to 0.2 g wet weight of organisms/ml of solution being preferred and 0.1 g of organisms/ml of solution being most preferred.

Any homogenizer is suitable, but optimum results are obtained with the type which is provided with two concentric teethed rings, whereby the forces created by one set of teeth pressing close to the other shears the organisms. Homogenizers which have been shown to be well suited for this purpose are the Ultraturrax Teckmar Tissumizer (Teckmar Corporation, Cincinnati, Ohio) or the Polytron (Brinkman Instruments, Westbury, New York). The temperature and duration of homogenization will depend in part on the type and speed at which the homogenizer is operated. However, the appropriate temperature/time combination avoids congealing of the lipopolysaccharides and denaturing of the protein while maximizing yields of protein I and minimizing contaminants. Temperatures in the range of $20^{\circ}-60^{\circ}C$ are suitable, the range of $35^{\circ}-50^{\circ}C$ being preferred and $45^{\circ}C$ being optimal. Depending upon the speed at which the above-described homogenizer is operated, the duration of homogenization is generally between 5 to 30 minutes, a period of 10 minutes seeming to be optimum at a temperature of $45^{\circ}C$. Shorter periods tend to lower the yields of protein I, while longer periods result in increased yields of the desired protein I, but at the expense of contamination with greater amounts of proteins other than protein I and with intracellular components of gonococci.

These improvements in the procedure to extract protein I from the gonococcal outer membrane are responsible for 50 to 100 fold higher yields of protein I, in an antigenic and immunogenic form, as compared to the procedure previously described in U.S. Patent 4,203,971.

Detergents, such as sodium deoxycholate, Sarkosyl, or zwitterionic agents may be utilized in the extraction-homogenization procedure to provide equally high or higher yields of protein I. However, the protein resulting therefrom is poorly antigenic, and non-immunogenic for humans, and is, therefore, useless as a source of protein I for human vaccine preparations. For this reason, all detergents are avoided during the initial homogenization of gonococci to extract protein I.

Following homogenization, the organisms are removed by two relatively low speed sequential centrifugations at 10,000 x g to 50,000 x g; the sequence of 12,000 x g for 10 minutes, and 30,000 x g for 20 minutes has been found to be quite effective. Alternatively, other suitable techniques such as filtration may be employed, and the supernatant liquid (or filtrate) containing the outer membrane fragments with protein I collected thereby are further treated by high speed centrifugation to collect the membrane pellet. Conditions of 100,000 x g for two to four hours, up to 505,000 x g for one hour may be used, with higher yields of protein I in shorter time periods obtained with the higher g force.

The outer membrane pellet containing protein I, contaminating proteins, and toxic lipopolysaccharide is then treated as described in U.S. Patent 4,203,971, to remove the toxic lipopolysaccharide. This consists of resuspending the pellet well into a solution of 0.5 to 5% sodium deoxycholate, preferably starting with a 3% solution, and diluting to a final concentration of 1.5% sodium deoxycholate. The final solution also has present glycine in a concentration of 25 to 75mM, preferably 35 to 60mM and most preferably 50mM as well as 1 to 10mM EDTA, preferably in the form of its di- or tetra-sodium salt, the disodium salt being most preferred and in a concentration of preferably 3 to 7mM, most preferably 5mM disodium EDTA, in the final volume of 3 to 30 ml per pellet. The solution with a concentration of 50mM glycine and 5mM disodium EDTA has a pH of 9. This resuspended and diluted pellet may then be processed by column chromatography as described in U.S. Patent 4,203,971. However, a faster process that also provides higher yields of protein I is to replace the column chromatography with centrifugation of the entire volume of resuspended pellet at elevated speeds such as between 100,000 to 505,000 x g, preferably the latter, for 1 hour. Lower centrifugation g forces (i.e., 100,000 x g) for longer time periods (i.e., 4 hours) may be used, with lower yields of protein I being realized. Under these conditions, the solubilized LPS and protein II remain in the supernatant liquid, and the only partially solubilized protein I, free of more than 95% of the toxic LPS, is pelleted to the bottom of the centrifuge tube. The supernate containing LPS and protein II is removed, and the protein I pellet is resuspended with homogenization into 30 ml of ethanolamine (preferably 0.15M to 0.20M). This resuspended pellet is again centrifuged at, preferably, 505,000 x g for 1 hour. This procedure serves to wash the protein I pellet, removing most of the coprecipitated sodium deoxycholate detergent, and some additional LPS. The pellet containing predominately protein I is resuspended in a small volume of ethanolamine and frozen at low temperature, preferably $-70^{\circ}C$ or below, until used further.

B.     Solubilization Procedure

The solubilization procedure, described in detail below, allows preparation of a solution having a high concentration of protein I in a soluble form capable of passing through a sterilizing filter. This allows combination of several different serotypes of protein I into a single "multivalent" vaccine, which is necessary to provide widespread protection against most gonococci since protection is serotype specific, and there are ten (10) different serotypes of protein I. By providing a vaccine with a sufficiently high concentration to combine several serotypes, the final concentration of each serotype of protein I remains high enough to supply an immunizing dosage to humans within a total volume sufficiently small to be tolerated by the human arm. This was not possible with previous methods, and this new development makes possible the preparation of highly concentrated solutions of protein I serotypes and also makes possible the preparation of a vaccine to protect against invasive gonococcal infection.

Pellets of purified protein I, as described above, were prepared for protein I of each serotype to be used in the vaccine lots. The antigenicity of each pellet was determined by first suspending it in one of the extraction solvents mentioned above in the purification and extraction procedure, within the specified concentration range. Ethanolamine at a concentration of 0.17M is most preferred. A zwitterionic detergent is also added at a concentration of 0.01% to 1.0% (weight/volume total solution), preferably 0.05% to 0.5%. A detergent known as "Zwittergent 3-14" (a trademark of Hoechst A.G. - distributed by Calbiochem-Behring, La Hoya, California, for N-tetradecyl-N,N-dimethyl-3-ammonio-1-propanesulfonate) is preferred at a concentration of 0.1% (weight/volume total solution). At this concentration of Zwittergent and 0.17M ethanolamine, the preferred solution has a pH equal to or greater than 8.5.

A bath sonicator is employed to provide gentle sonication of the mixture for a period of 0.5 to 10 minutes, preferably for 1 to 3 minutes. The preparations are then centrifuged at low to moderate speeds, for a period of between 10 minutes and 1 hour, preferably at 30,000 x g for 20 minutes. A test for antigenicity, the ELISA test, is then performed on the supernatant liquid, after determination of the protein concentration of each as described by T.M. Buchanan and J.F. Hildebrandt in "Antigen-Specific Serotyping of Neisseria gonorrhoeae: Characterization Based Upon Principal Outer Membrane Protein", Infection and Immunity, 32:985-994 (1981). Essentially the ELISA test is performed as follows. Polystyrene tubes (13 by 75 mm) or microtiter plates, are coated with 1 ml (or 0.1-0.2 ml for plates) of a 2 µg/ml concentration of different protein I antigens at 37°C for 5 h in 0.06M NaHCO, buffer pH 9.5 or Tris pH 8.0. These coated tubes are then stored at 4°C until used in the assay with human or rabbit sera to measure antibody to protein I antigen or used for inhibition assays to quantitate protein I antigen on the surface of gonococci. The protein I coated tubes retain antigenicity for more than 1 year when stored at $4^{\circ}$C with the 2 µg/ml protein I coating solution. To conduct the assay, the tubes are emptied and washed three (3) times with phosphate-buffered saline containing 0.05% Tween 20 (PBST) and then incubated for 30 min at $37^{\circ}$C with the serum dilution, washed three (3) times with PBST, incubated for 30 min at $37^{\circ}$C with a 1:2,500 to 1:15,000 dilution (depending upon the activity of a given lot) of the immunoglobulin G goat anti-human or goat anti-rabbit antibody linked to horseradish peroxidase, washed three (3) times with PBST, and incubated at room temperature in the dark for 30 min with the substrate $H_2O_2$ and the chromagen o-phenylenediamine. The reactions are stopped by adding 125 µl of 8 N $H_2SO_4$, and the optical density is read at 490 nm. Antibody to protein I is quantitated by the optical density produced by bound enzyme that develops color from substrate and chromagen.

Amounts of protein I antigen are quantitated for each serotype standard strain by comparing the inhibition of optical density produced by a known quantity of organisms with that produced by known weights of purified protein I antigen. For routine testing, a strain is preincubated for 15 min at $37^{\circ}$C with a constant amount of antiserum. A strain is accepted as a given serotype if this antiserum-organism mixture produces 30% reduction in the optical density in the ELISA test when added to protein I-coated tubes, as compared with the optical density of an ELISA performed identically except that organisms are omitted. Alternatively, purified protein I may be substituted for whole organisms in the ELISA, and its antigenicity may be characterized by the amount of inhibition produced in the ELISA by a given weight of protein I.

The preparations demonstrating the highest protein I concentrations and antigenicity are then pooled, either before or after dialysis against the solvent to remove detergent. The dialysis baths are changed frequently and non-pyrogenic water is used to prepare the last few dialysis bath changes of the solvent, preferably 0.15M ethanolamine.

The presence of micellar zwitterionic detergent is tested for by a suitable test procedure. When Zwittergent (3-14) is employed as the detergent, a sensitive resin assay, comprised of a suitable azo dye in an inert carrier that is solubilized in the presence of detergent micelles, available from Calbiochem-Behring Corporation, provides a sensitivity down to 0.005% by weight of Zwittergent. Recommended procedure also includes testing for the presence of deoxycholate buffer with 80% $H_2SO_4$ at an optical density of 425 nm.

Solutions which demonstrate the absence of zwitterionic detergent and deoxycholate are then filtered through a bacteria filtering membrane such as a 0.22 μm Millipore filter. The vaccine is further modified to improve storage and administration to humans by adding non-pyrogenic water, a preservative such as thimerosal (also known as merthiolate or sodium ethylmercurithio-salicylate), an adjuvant such as $Al(OH)_3$ gel and 6M HCl to reduce the pH if necessary. It has been found that the solubility of the protein I increases with pH, probably because the extracting or solubilizing solvent, particularly ethanolamine is more stable at higher pH. Thus, a pH of 8.5 to 11.5 is suitable and values of 9 to 10.5 are preferred.

The following examples are provided to illustrate how the present invention may be carried out, including some of its preferred embodiments. It is to be understood, however, that the instant invention is not to be limited to or by what is disclosed in these examples.

Example 1

Preparation Of Purified Serotypes
Of N.g. Protein I

Neisseria gonorrhoeae were grown for 20 hours on solid GC agar base media containing 1% defined supplement at 36.5°C in the

presence of 5% $CO_2$. The organisms were suspended in ice cold 0.15M NaCl and centrifuged at 12,000 x g for 10 minutes. The pellet formed thereby, weighing 30 g, was combined with 300 ml of buffering solution composed of 0.17M 2-aminoethanol and 0.02M disodium EDTA, and the mixture was introduced into an Ultra-turrax Teckmar Tissumizer. After homogenization of the organism pellet in 0.17M ethanolamine and 0.02M disodium EDTA (0.1 g wet weight organisms/ml) for 10 minutes at 45°C, the organisms were removed by two (2) sequential centrifugations at 12,000 x g for 10 minutes. The supernate containing outer membrane fragments was then centrifuged in a 70 Ti rotor (Beckman) at 505,000 x g for 1 hour to collect the membrane pellet. Each membrane pellet was resuspended and dissolved with homogenization using an Ultra-turrax Teckmar Tissumizer (Teckmar Co., Cincinnati, Ohio) into 3.0% sodim deoxycholate and diluted to a final concentration of 1.5% sodium deoxycholate, 50mM glycine, 5mM di-Na EDTA (pH 9.0, 30 ml) and recentrifuged at 505,000 x g x 1 hour. The supernate was retained to use for subsequent purification of LPS and protein II, and the pellet containing the protein I for use in the vaccine was resuspended by homogenization with the Ultra-turrax homogenizer into 30 ml of 0.17M ethanolamine. In all cases, the ethanolamine used for these vaccine preparations was redistilled twice to remove any non-ethanolamine contaminants prior to its usage. This suspension was again centrifuged at 505,000 x g for 1 hour to collect the pellet, which was then resuspended in a small volume ethanolamine and frozen at -70°C until needed. Similar pellets were prepared for protein I of each serotype used in the vaccine lots.

## Example 2

Determination Of Protein I Concentration
And Antigenicity Of Each Pellet

The antigenicity of each pellet was determined by first suspending it in 0.17M ethanolamine, 0.1% Zwittergent (3-14), using gentle sonication in a bath sonicator for 1-3 minutes. These preparations were centrifuged at 30,000 x g x 20 minutes and the protein concentration of the supernate of each preparation was determined, and its ability to inhibit an ELISA test based upon that protein I serotype was determined. The protein I preparations with the best antigenicity on a protein weight basis were pooled and utilized for the vaccine lots. A lot identified as Lot 12 was formed from 180 mg each of protein I serotypes 1, 5 and 9, and a lot identified as Lot 14 was composed of protein I from serotypes 1 (120 mg), 5 (110 mg), 7 (120 mg), 8 (120 mg), and 9 (70 mg).

Example 3

Solubilization Procedure

The volume of Lot 12 was adjusted to 150 ml with 0.17M ethanolamine, reducing the Zwittergent concentration to 0.045%, and dialyzed for 5 days at $4^{o}$C against 0.15M ethanolamine with frequent changes of the dialysis bath. Non-pyrogenic water was used to prepare the last two dialysis bath changes of 0.15M ethanolamine. After dialysis, the volume was 168 ml, the protein concentration was 3.2 mg/ml, and there was no micellar Zwittergent present in the vaccine lot as tested by a sensitive resin assay provided by Calbiochem-Behring Corporation that is sensitive to concentrations of 0.005% Zwittergent. This assay consists of adding 5 mg resin to 1 ml of test sample. This preparation is incubated at room temperature for 15 minutes, with mixing at 5 minute intervals. The mixture is then centrifuged, spun at 10,000 rpm for 1 minuted in a microfuge B (Beckman) to remove the resin. The optical density of the supernate is read at 525 nm. There was also no deoxycholate detectable in the dialyzed vaccine, as tested by an assay using 80% $H_2SO_4$ at optical density of 425 nm. This assay is sensitive to 0.001% concentrations of sodium deoxycholate. This dialyzed Lot 12 preparation was filter sterilized through a 0.22 micron Millipore filter, after first rinsing the filter with 10 cc of 0.15M ethanolamine in non-pyrogenic water. The pH of this sterile product was then reduced to 9.5 with sterile 6M HCl. Aliquots of this final preparation were removed for further testing in Washington State, and the remaining vaccine was sent to the Michigan State Department of Health Laboratories for a bulk sterility test, packaging and safety, sterility and pyrogenicity tests on the packaged product. The Lot 14 vaccine was prepared identically to Lot 12 except that it contained 5 protein I serotypes as noted above, and was diluted to a final concentration of 0.05% Zwittergent prior to dialysis. No Zwittergent or sodium deoxycholate could be detected in Lot 14 after dialysis and filtering as tested by the methods described above, and the final pH was 9.6. The final protein concentration of Lot 12 after filtration was 2.7 mg/ml and of Lot 14 was 4.3 mg/ml. Assuming no selective loss of one protein I serotype as compared to another, Lot 12 contains 0.9 mg/ml each of serotypes 1, 5 and 9. With the same assumption, the protein I concentrations by serotype for Lot 14 are 0.95 mg/ml (serotypes 1, 7 and 8), 0.88 mg/ml (serotype 5), and 0.57 mg/ml (serotype 9).


## Example 4


### Identity Of Vaccine Lots 12 and 14

Each vaccine lot was evaluated by sodium dodecyl sulfate polyacrylamide slab gel and the gel was stained with Coomassie Blue and subsequently with Stains-All. The gel indicated that each vaccine lot was primarily protein I of varying molecular weights (34,000-39,000), corresponding to the serotypes that it contains, with small amounts of contaminating protein II (24,000-28,000), III (30,000), and higher molecular weight proteins. There were also trace amounts of lipopolysaccharide present in each vaccine preparation. By KDO determinations, this was quantitated as 3.5% of the protein in Lot 12, 4% of the protein in the Lot 14 vaccine. In addition to the above components, the vaccine contained 0.15M twice redistilled ethanolamine, a normal component found in the human body, and pyrogen-free water. The pH was 9.5 and there was no merthiolate or other preservative.

## Example 5

### Potency - Lots 12 and 14

Each vaccine lot was injected into one rabbit without adjuvant in a dosage of 50 micrograms protein per serotype, given twice, a month apart, followed by a third injection one week after the first booster. Each vaccine produced a good immune response to each serotype, in the rabbit, as measured by ELISA.

In addition, ELISA tests were used to evaluate the antigenicity of each serotype in the final vaccine preparation. In each case, the vaccine lots were compared to a standard highly antigenic preparation of each serotype. These results indicated that the antigenicity of serotypes 1, 5 and 9 in Lot 12 was comparable to the standard for these protein I serotypes. For Lot 14, antigenicity was also comparable to the standard for all five serotypes, 1, 5, 7, 8 and 9. Each vaccine lot was capable of inhibiting the ELISA with 0.0005 to 0.005 mg for each serotype of protein I.

These two vaccine lots have been tested for safety and immunogenicity in a small number of human volunteers, using a protocol and consent form approved by the University of Washington Human Subjects Committee. Each vaccine lot is safe when injected subcutaneously in dosages of 50 micrograms to 200 micrograms of protein per serotype. The side effects of the vaccine were confined to local redness, swelling and tenderness at the site of the injection. Higher dosages occasionally produce transient fever in the recipient. Subjects receiving the vaccine responded with increased antibody to each of the protein I serotypes contained in the vaccine. The antibody was capable of killing gonococci of the same protein I serotype in a complement-dependent, antibody-mediated bactericidal reaction. It is thus expected that recipients of these vaccines can be protected against invasive gonococcal infection (P.I.D. or D.G.I.) produced by N.g. organisms bearing protein I on their surfaces, of the same protein I serotypes as contained in the vaccine.

WHAT IS CLAIMED IS

1. A protein I vaccine capable of conferring a protective immune response in mammals to <u>Neisseria gonorrhoeae</u> having protein I as the principal outer membrane protein comprising at least one protein I serotype, each of said serotypes having a solubility of from about 5 to about 10 mg/ml.

2. A vaccine according to Claim 1, wherein the total protein concentration of the vaccine is at least 5 mg/ml.

3. A vaccine according to Claims 1 or 2, wherein the vaccine contains from about 0.5 mg/ml to about 10 mg/ml protein per serotype.

4. A vaccine according to Claims 1, 2 or 3, wherein the mammal is a human, and the vaccine comprises at least one serotype in an amount of at least about 100 $\mu$g per serotype.

5. A vaccine according to Claims 1, 2, 3 or 4, wherein said protein I includes subunits having molecular weights of from 34,000 to 39,000 Daltons.

6. A vaccine according to Claims 1, 2, 3 or 4, wherein the vaccine contains from 3 to 9 serotypes.

7. A vaccine according to Claims 1, 2, 3 or 4, wherein the vaccine contains a single protein I serotype capable of conferring a protective immune response to <u>Neisseria gonorrhoeae</u> causing disseminated gonoccocal infection.

8. A vaccine according to Claims 1, 2, 3 or 4, wherein the vaccine contains five protein I serotypes capable of conferring a protective immune response to <u>Neisseria</u>

gonorrhoeae causing gonoccocal salpingitis.

9.   A vaccine according to Claims 1-8, wherein each of the serotypes present is highly antigenic and capable of inhibiting binding of the labelled standard antigen to a serotype-specific antiserum in a standardized competitive immunoassay in an amount no greater than about 0.005 mg.

10.   A method for extracting and recovering protein I from Neisseria gonorrhoeae organisms in which protein I is the principle outer membrane protein comprising homogenizing Neisseria gonorrhoeae organisms at a temperature of at least about 20°C in the presence of an extraction solvent comprising ethanolamine which interacts with the protein I present to increase the solubility thereof, and a chelating agent which binds divalent cations and destabilizes the gonoccocal outer membrane, and recovering a purified protein I product comprising outer membrane fragments and associated protein I in an amount of from about 5 to 10 mg of protein I per gram wet weight of the starting organisms.

11.   A method according to Claim 10, wherein the concentration of said ethanolamine is 0.05M to 0.6M.

12.   A method according to Claim 10, wherein said chelating agent is EDTA, the disodium salt of EDTA, or the tetrasodium salt of EDTA.

13.   A method of preparing highly purified protein I according to Claims 10, 11 or 12, wherein recovery of the purified protein I product is accomplished by centrifugation.

14.   A method of preparing a vaccine capable of conferring a protective immune response in mammals to Neisseria gonorrhoeae microorganisms comprising the sequential steps of:

(a)   preparing at least one purified serotype of protein I;

(b)   suspending the purified protein I in a solution comprising an organic extraction solvent and a zwitterionic detergent to solubilize protein I;

(c)   separating undissolved protein I and detergent from dissolved protein I and recovering solubilized protein I; and

(d)   preparing a vaccine from the recovered solubilized protein I.

15.   The method of Claim 14, wherein sonication of the suspended purified protein precedes separation of undissolved protein I.

16.   The method of Claims 14 or 15, wherein the organic extraction solvent is ethanolamine, diethanolamine, triethanolamine, 2-amino-1-propanol, 2-amino-1, 3-propanediol,          or tris (hydroxymethyl) aminomethane.

17.   The method of Claims 14 or 15, wherein the organic extraction solvent is ethanolamine.

18.   The method of Claims 14-17, wherein the concentration of the organic extraction solvent is between 0.05M and 0.6M.

19.   The method of Claims 14-18, wherein the zwitterionic detergent is N-tetradecyl-N, N-dimethyl-3-ammonio-1-propanesulfonate.

20.   The method of Claims 14 or 15, wherein each of the purified serotypes of protein I is separately suspended in aqueous solutions containing ethanolamine and N-tetradecyl-N, N-dimethyl-3-ammonio-1-propanesulfonate, and are combined after separation from detergent and undissolved protein I.

21. The method of Claims 14 or 15, wherein the recovered solubilized protein I is passed through a bacteria separating filter.

22. The method of Claim 20, wherein the final vaccine comprises from 3 to 9 serotypes.

23. A method of protecting humans against infection by Neisseria gonorrhoeae microorganisms comprising administering an immunologically effective amount of the vaccine according to Claims 26- 37 or Claims 1-9.

24. A method for the recovery of highly soluble, highly antigenic, and highly immunogenic form of protein I from Neisseria gonorrhoeae comprising solubilizing protein I purified with respect to associated toxic lipopolysaccharides in aqueous suspension with a zwitterionic detergent and an organic solvent which interacts with protein I, and recovering a highly immunogenic and antigenic form of solubilized protein I having a solubility of about 5 to about 10 mg/ml.

25. The method of Claim 24, wherein the protein I suspension is sonicated during the solubilization step.

26. The method of Claim 24, wherein the recovered solubilized protein I is sufficiently soluble to pass through a sterilizing microfilter in concentrations of from about 5 to about 10 mg/ml.